(19) Europäisches Patentamt / European Patent Office / Office européen des brevets

(11) **EP 3 127 866 A1**

(12) **EUROPEAN PATENT APPLICATION**
published in accordance with Art. 153(4) EPC

(43) Date of publication:
08.02.2017 Bulletin 2017/06

(51) Int Cl.:
*C01B 39/16* (2006.01)        *B01D 69/12* (2006.01)
*B01D 71/02* (2006.01)        *B01J 20/18* (2006.01)
*B01J 20/30* (2006.01)        *C01B 39/24* (2006.01)
*C01B 39/38* (2006.01)        *C01B 39/40* (2006.01)
*C01B 39/48* (2006.01)

(21) Application number: 15772223.2

(22) Date of filing: 20.03.2015

(86) International application number:
PCT/JP2015/058449

(87) International publication number:
WO 2015/151854 (08.10.2015 Gazette 2015/40)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA

(30) Priority: 01.04.2014 JP 2014075557

(71) Applicant: NGK Insulators, Ltd.
Nagoya-shi, Aichi 467-8530 (JP)

(72) Inventors:
• NODA, Kenichi
  Nagoya-shi
  Aichi 467-8530 (JP)
• HAGIO, Takeshi
  Nagoya-shi
  Aichi 467-8530 (JP)

(74) Representative: Teipel, Stephan et al
Lederer & Keller
Patentanwälte Partnerschaft mbB
Unsöldstrasse 2
80538 München (DE)

(54) **ZEOLITE, SEPARATION MEMBRANE STRUCTURE, AND PROCESS FOR PRODUCING ZEOLITE**

(57) A zeolite includes Si, Al, Ag and at least one of an alkali metal or alkaline earth metal, and satisfies the relation $0.02 \leq Ag[mol\%]/(Si[mol\%] + 10 \times T[mol\%]) \leq 0.17$ (wherein, $T[mol\%]$ denotes the molar concentration of the alkali metal and alkaline earth metal.)

FIG. 4

EP 3 127 866 A1

**Description**

TECHNICAL FIELD

The present invention relates to a zeolite, a separation membrane structure, and to a method of manufacturing the zeolite.

BACKGROUND ART

A method is known to typically separate one of an olefin and a paraffin from a mixture thereof by use of an Ag-Y type zeolite membrane produced by ionic exchange of an Na-Y type zeolite membrane.

[Citation List]

[Patent Literature]

[Non-Patent Literature 1] Masahiko MATSUKATA, et al. (two others), "Gin Kachion Kokankei Y-kei Zeoraitomaku ni yoru Puropan/Puropiren Bunri no Kanosei" March 17 to 19, 2013, SCEJ (The Society of Chemical Engineers Japan) 78th Annual Meeting Q109 (Searched February 24, 2014) Internet "URL:http://www3.scej.org/meeting/78a/prog/sess_22.html"

SUMMARY OF THE INVENTION

Technical Problem

However, the method in Non-Patent Literature 1 is configured for Ag ionic exchange by immersion of an Na-Y type zeolite in a silver nitrate aqueous solution, and therefore are large portion of the $Na^+$ is exchanged by $Ag^+$. As a result, ionic Ag undergoes agglomeration and forms metallic Ag which over time causes the problem that there is not a sufficient function of separation of an olefin and paraffin.

The present invention is proposed in light of the situation described above, and has the purpose of providing a zeolite that exhibits a stable separation function for an olefin and paraffin, a separation membrane structure and a method of producing the zeolite.

Solution to Problem

The zeolite of the present invention includes Si, Al, Ag, and at least of one of an alkali metal and an alkaline earth metal, and satisfies Relation 1 below.

$0.02 \leqq Ag[mol\%]/(Si[mol\%]+10xT[mol\%]) \leqq 0.17$ (wherein, T[mol%] denotes the molar concentration of the alkali metal or the alkaline earth metal.)

Effect of Invention

The present invention enables the provision of a zeolite that exhibits a stable separation function for an olefin and paraffin, a separation membrane structure and a method of producing the zeolite.

BRIEF DESCRIPTION OF THE DRAWINGS

FIG. 1 is a perspective view illustrating a separation membrane structure.
FIG. 2 illustrates a sectional view of A-A of FIG. 1.
FIG. 3 illustrates a sectional view of B-B of FIG. 2.
FIG. 4 illustrates an enlarged plane view of the portion X of FIG. 3.
FIG. 5 illustrates an enlarged sectional view of the portion X of FIG. 3.

DESCRIPTION OF EMBODIMENTS

Next, the embodiments of the present invention will be described making reference to the figures. In the following embodiments, a configuration will be described in which a zeolite according to the present invention is applied to a zeolite membrane of a separation membrane structure. In the description of the figures below, the same or similar portions are denoted by the same or similar reference numerals. However, the figures are merely illustrative and the ratio of respective dimensions or the like may differ from the actual dimensions. Therefore, the actual dimensions or the like should be determined by reference to the following description. Furthermore, it goes without saying that the ratios or the relations of dimensions used in respective figures may be different.

Structure of Separation Membrane Structure 100

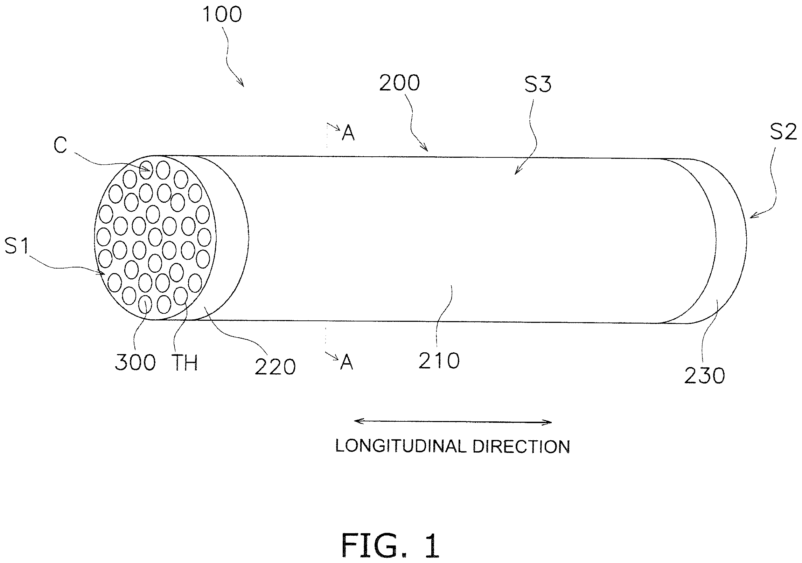

FIG. 1 is a perspective view illustrating a separation membrane structure 100.

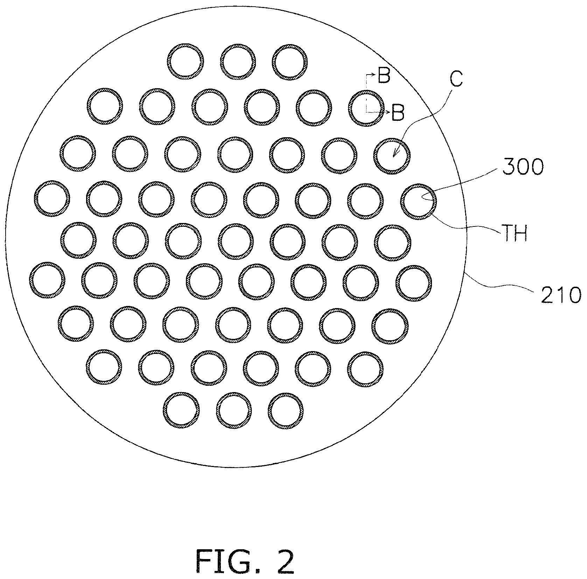

FIG. 2 illustrates a sectional view of A-A of FIG. 1.

The separation membrane structure 100 includes a substrate 200 and a zeolite membrane 300.
The substrate 200 includes a substrate main body 210, a first seal portion 220, and a second seal portion 230.
The substrate main body 210 is configured as a porous body. The substrate main body 210 is formed as a circular cylinder. The length of the substrate main body 210 in a longitudinal direction is 150 to 2000 mm, and the diameter of the substrate main body 210 in the short width direction is 30 to 220 mm. However there is no limitation in this regard. The substrate main body 210 is configured by a porous material. The porous material that forms the substrate main body 210 includes use of a ceramic, metal, resin, or the like. In particular, use of a porous ceramic is preferred. The aggregate particles used in relation to the porous ceramic material include alumina ($Al_2O_3$), titania ($TiO_2$), mullite ($Al_2O_3 \cdot SiO2$), potsherd, and cordierite ($Mg_2Al_4Si_5O_{18}$), or the like, and in particular, alumina is preferred in light of ease of availability, formation of a stable clay, and anticorrosive properties.
The substrate main body 210 may include an inorganic binder in addition to the porous material. The inorganic binder may include at least one of titania, mullite, sinterable alumina, silica, glass frits, clay minerals, and sinterable cordierite. The porosity of the substrate main body 210 may be configured to 25% to 50%. The average pore diameter of the substrate main body 210 may be 0.05 micrometers to 25 micrometers. The average particle diameter of the porous material that configures the substrate main body 210 may be 0.1 micrometers to 100 micrometers.
In the present embodiment, the term "average particle diameter" denotes the value of the arithmetic mean for the maximum diameter of 30 measured particles that are measured by cross sectional micro-structure observation by use of a scanning electron microscope (SEM).
The substrate main body 210 has a first end surface S 1, a second end surface S2, a side surface S3, and plural through holes TH. The first end surface S1 is provided opposite to the second end surface S2. The side surface S3 is connected to the outer edge of the first end surface S 1 and the second end surface S2. The through holes TH pass through from the first end surface S 1 to the second end surface S2. The sectional shape of through holes TH is circular, however there is no limitation in this regard. The inner diameter of the through holes TH is 1 mm to 5 mm
The first seal portion 220 covers the entirety of the first end surface S1 and a portion of the side surface S3. The first seal portion 220 inhibits infiltration, from the first end surface S 1 to the substrate main body 210, of the mixed fluid to be filtered. The first seal portion 220 is formed so that a barrier is not formed in relation to the input port for cells C as described below. The material that configures the first seal portion 220 includes use of glass, metal, or the like. Glass is preferred in light of adaptability with the thermal expansion coefficient of the substrate main body 210.
The second seal portion 230 covers the entirety of the second end surface S2 and a portion of the side surface S3. The second seal portion 230 inhibits the infiltration, from the second end surface S2 to the substrate main body 210, of the mixed fluid. The second seal portion 230 is formed so that a barrier is not formed in relation to the input port for the cells C. The second seal portion 230 may be configured by use of the same material as the first seal portion 220.
The zeolite membrane 300 is formed on an inner surface of the through holes TH of the substrate main body 210. In this configuration, the separation membrane 300 is formed as a cylindrical tube. The zeolite membrane 300 has a crystal structure such as FAU (Y-type, X-type), LTA (A-type), LTL (L-type), MFI, MEL, DDR, MOR, FER, CHA, BEA, CON, MSE, MWW, or the like. The inner side of the zeolite membrane 300 forms a cavity (referred to below as a "cell C") for allowing passage of the mixed fluid to be filtered. In the present embodiment, a mixed fluid of an olefin and paraffin is assumed to be the object of filtration. The zeolite membrane 300 has a function of selectively separating one of an olefin and paraffin.

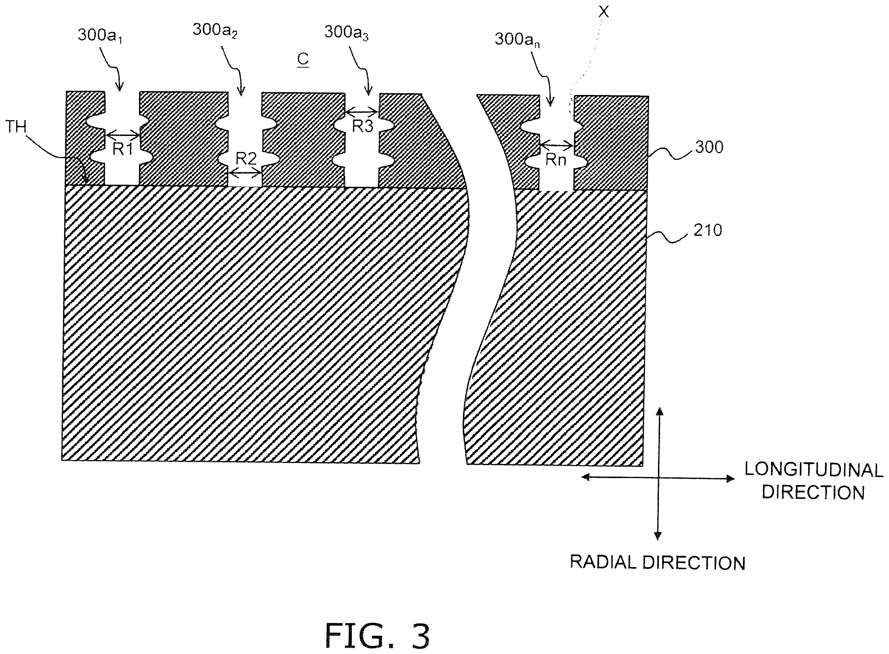

FIG. 3 illustrates a sectional view of B-B of FIG. 2. The zeolite membrane 300 includes a plurality of pores $300a_1$ to $300a_n$ (referred to below as "pores 300a" for the sake of convenience). The inner diameter R1 to Rn of the respective pores 300a may differ in each pore. The inner diameter R 1 to Rn is the smallest diameter of each pore and is the short radius when there is a long radius and a short radius in the pore.
At least one of the inner diameter R1 to Rn is preferably greater than or equal to 0.4 nm. That is to say, the maximum value of the inner diameter R1 to Rn is preferably at least 0.4 nm. A configuration in which the maximum value of the inner diameter R1 to Rn is at least 0.4 nm enables suppression of reductions in the olefin permeation rate. The respective inner diameter R1 to Rn is preferably less than or equal to 0.7 nm. That is to say, the maximum value of the inner diameter R1 to Rn is preferably no more than 0.7 nm. A configuration in which the maximum value of the inner diameter R1 to Rn is no more than 0.7 nm enables suppression of reductions in olefin selectivity.

The inner diameter R1 to Rn of pores 300a in the zeolite membrane 300 is determined uniquely by the framework structure of the zeolite membrane 300. The inner diameter R1 to Rn of each framework structure may be obtained by use of the values disclosed in The International Zeolite Association (IZA), "Database of Zeolite Structures" [online], [searched February 13, 2015], Internet URL: http://www.iza-structure.org/databases/. Framework structures for the zeolite membrane 300 that satisfy a maximum value of the inner diameter R1 to Rn of at least 0.4 nm to no more than 0.7 nm include for example LTA (A-type), MFI, MEL, MOR, FER, BEA, CON, MSE, MWW, or the like.

From the point of view of olefin permeation rate, the thickness of the zeolite membrane 300 in a radial direction is preferably no more than 10 micrometers, and more preferably no more than 3 micrometers. When the thickness of the zeolite membrane 300 is configured to be no more than 10 micrometers, it is possible to suppress a reduction in the olefin permeation rate.

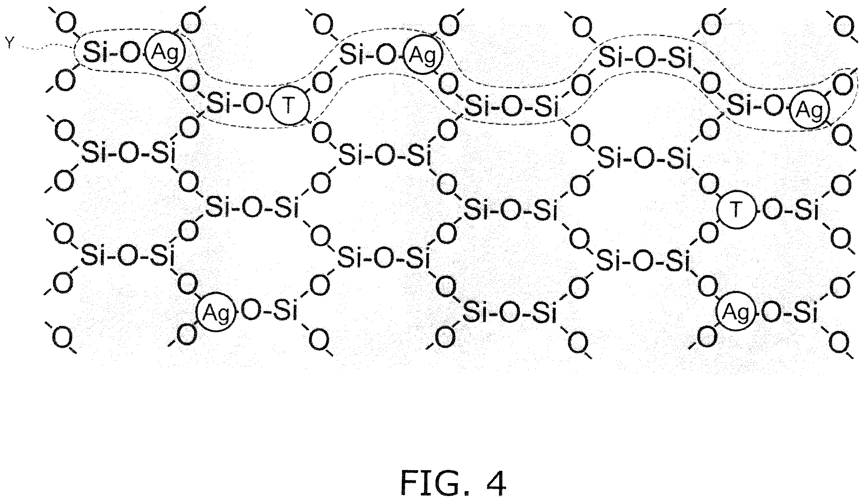

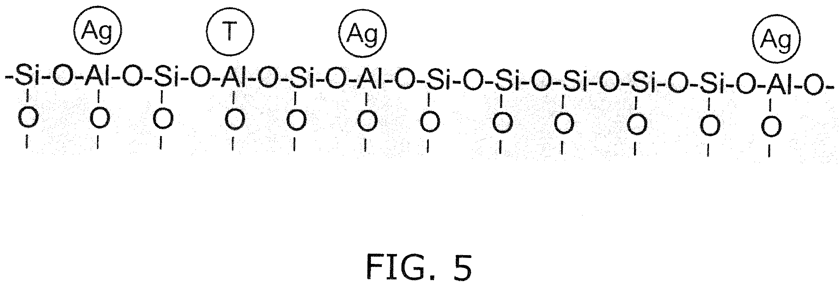

FIG. 4 illustrates an enlarged plane view of the portion X of FIG. 3 shown by the broken line. FIG. 5 illustrates an enlarged sectional view of the portion Y of FIG. 4 shown by the broken line. Although FIG. 4 and FIG. 5 show the three dimensional atomic arrangement in a two dimensional configuration, Si that is a tetrad and Al that is a triad respectively form four covalent bonds. FIG. 4 and FIG. 5 illustrate a schematic atomic arrangement, and the actual three dimensional atomic arrangement is determined by the framework structure of the zeolite membrane 300.

The zeolite membrane 300 includes Si, Al, Ag, and at least one of an alkali metal and an alkaline earth metal. The alkali metal includes Li, Na, K, Rb, Cs and Fr. The alkaline earth metal includes Mg, Ca, Sr, Ba, and Ra. In FIG. 4 and FIG. 5, the alkali metal ion and alkaline earth metal ion is denoted as "T".

The zeolite membrane 300 has a structure in which oxygen is shared at the apex of a $SiO_4$ tetrahedron and an $AlO_4$ tetrahedron, and is connected in a three dimensional configuration. Ionic Ag or T is contained in the pores 300a in order to maintain a charge balance due to the presence of Al that is a lower valency atom than Si. Ag ions exhibit a greater tendency to form complexes with an olefin than with paraffin, and therefore an olefin can migrate in the pores 300a while repeating formation of a complex with Ag ions and disassociation from Ag ions. Paraffin cannot flow into the pores 300a due to the presence of olefin. As a result, the zeolite membrane 300 exhibits olefin/paraffin selectivity. In the present embodiment, Ag ions are separated by an interval and T is disposed between two Ag ions. As a result, formation of metallic Ag induced by agglomeration of ionic Ag is inhibited.

In this context, the Ag proximity AD is given by the following formula (1) that uses a molar ratio.

$$\text{Ag proximity AD} = \text{Ag [mol\%]} / (\text{Si [mol\%]} + 10 \times \text{T [mol\%]}) \qquad \ldots(1)$$

T [mol%] denotes the total molar concentration of the alkali metal and alkaline earth metal contained in the zeolite membrane 300.

Although an agglomeration inhibition effect on Ag ions when T is disposed between two Ag ions is difficult to derive theoretically, the results of experimental investigation by the present inventors confirm that an Ag agglomeration inhibition effect equivalent to disposing 10 Si elements between two Ag ions is enabled by disposing one T between two Ag ions. Ag proximity AD shown by Formula (1) has been derived based on the above assumptions.

Ag proximity AD shown by Formula (1) is preferably greater than or equal to 0.02 and less than or equal to 0.17, and more preferably greater than or equal to 0.03 and less than or equal to 0.14.

The separation membrane structure 100 that comprises the zeolite according to the present invention preferably exhibits selectivity that expresses a separation function in relation to olefin/paraffin of at least 2, more preferably at least 5, and still more preferably at least 10. Selectivity is calculated as shown below. A pure olefin gas or a pure paraffin gas is used so that the gas is absorbed in a zeolite powder using measurement conditions of 23 degrees C and 0 MPa to 1 MPa. The result of repeating absorption 10 times is used calculate (olefin absorption amount at 1 MPa) / (paraffin absorption amount at 1 MPa) as selectivity (olefin selectivity). The gas separation function is enhanced as selectivity increases. The pure olefin gas may be ethylene, propylene, or the like. The pure paraffin gas may be methane, ethane, propane, or the like.

Method of Manufacturing Separation Membrane Structure 100

Firstly, a green body for the substrate main body 210 that includes plural through holes TH is formed using clay that includes a porous material. The method of forming the green body for the substrate main body 210 includes use of an extrusion molding method using a vacuum extrusion molding device, in addition to a press molding method or a slip cast method.

Next, the green body for the substrate main body 210 is fired (for example, 1000 to 1600 degrees C, 1 to 50 hours) to thereby form the substrate main body 210.

Next, glass is coated and fired (800 to 900 degrees C) onto both ends of the substrate main body 210. In this manner, the first seal portion 220 and second seal portion 230 are formed, and the substrate 200 is completed.

A zeolite membrane 300 is formed on an inner surface of each through hole TH using the following process.

Firstly, a zeolite powder (zeolite seed crystal) is deposited on an inner surface of the through hole TH. Next, a starting material solution is prepared by mixing a silica source, an alumina source and an alkali source in a solvent. The starting material solution may include addition of a structure regulation agent as required.

The silicon source includes colloidal silica, tetraethoxysilane, water glass, silicon alkoxide, fumed silica, precipitated silica, or the like. The source of alumina includes salts of aluminum such as aluminum hydroxide, sodium aluminate, aluminum sulfate, aluminum nitrate, aluminum chloride, or the like, in addition to alumina powder, colloidal alumina, or the like. The source of alkali includes alkali metals such as sodium hydroxide, lithium hydroxide, potassium hydroxide, or the like, and alkaline earth metals such as magnesium hydroxide, calcium hydroxide, or the like. When there is a need for inclusion of an alkali metal or alkaline earth metal in the compounds being the source of silica or the source of alumina, separation addition of the alkali source is not required.

The solvent includes water or the like. The structure regulation agent includes organic compounds such as tetraethylammonium hydroxide, tetraethylammonium bromide, 1-adamantane amine, tetrapropylammonium hydroxide, tetrapropylammonium bromide, tetramethylammonium hydroxide, or the like. The molar ratio of the structure regulation agent relative to the silica source may be configured as 0.03 to 0.4, and the molar ratio of the solvent relative to the silica may be configured as 50 to 500. A fluorine source such as hydrofluoric acid or the like may be included in the starting material solution.

Hydrothermal synthesis is performed by immersing the substrate 200 in a pressure resistant container containing the starting material solution. The synthesis temperature may be 90 to 200 degrees C, and the synthesis time may be 0.5 to 300 hours.

Next, the substrate 200 including formation of the zeolite membrane is washed in water or hot water (40 to 100 degrees C) and dried at 70 to 100 degrees C.

When the zeolite membrane contains structure regulation agent, the pores 300a are formed by placing the substrate 200 including formation of the zeolite membrane into an electrical furnace and heating in an atmosphere of air (400 to 800 degrees C, 1 to 200 hours) to thereby combust and remove the structure regulation agent.

Next, the zeolite membrane is brought into contact with the mixed fluid (0.01 to 1 mol/L) of at least the silver compound and the alkali metal compound and/or alkaline earth metal compound to thereby cause ion exchange of the alkali metal and/or alkaline earth metal in the zeolite membrane with Ag. The mixed fluid may contain at least one of the alkali metal compound and/or alkaline earth metal compound. The mixed fluid for example may be prepared by mixing an aqueous solution including the alkali metal compound and/or alkaline earth metal compound with an aqueous solution containing the silver compound. In this context, since the mixed fluid contains the alkali metal compound and/or alkaline earth metal compound in addition to Ag, not all the alkali metal and/or alkaline earth metal in the zeolite is subjected to ion exchange with Ag, and there is residual alkali metal and/or alkaline earth metal in the zeolite.

The mixing ratio of the silver compound and the alkali metal compound and/or alkaline earth metal compound may be set with reference to Formula (1) above. The silver compound includes silver nitrate, silver perchlorate, silver acetate, silver tetrafluoroborate, silver hexafluorophosphate, complexes such as silver diamine, or the like. Alkali metal compounds or alkaline earth metal compounds include hydroxides, chlorides, nitrates, sulfates, fluorides, acetates, fluoro complexes, ammine complexes, or the like of alkali metal or alkaline earth metal. The mixed solution may comprise components other than silver compounds, alkali metal compounds, and alkaline earth metal compounds.

As described above, a separation membrane structure 100 that includes a substrate 200 and a zeolite 300 is completed. The method of producing the zeolite membrane 300 may be adapted to the preparation of a zeolite membrane having a crystal structure such as FAU (Y-type, X-type), LTA (A-type), LTL (L-type), MFI, MEL, DDR, MOR, FER, CHA, BEA, CON, MSE, MWW, or the like.

OTHER EMBODIMENTS

Although an embodiment of the present invention has been described, the present invention is not limited to the above embodiment, and various modifications are possible within a scope that does not depart from the spirit of the invention.

(A) In the above embodiment, the substrate 200 is configured to include the substrate main body 210, the first seal portion 220, and the second seal portion 230. However, a configuration is possible that includes only one of the first seal portion 220 and the second seal portion 230 or neither the first seal portion 220 nor the second seal portion 230.

(B) In the above embodiment, although the first seal portion 220 and the second seal portion 230 cover a portion of the side surface S3 of the substrate main body 210. However, the side surface S3 of the substrate main body 210 need not be covered.

Although particular reference was not made in the above embodiment, the substrate 200 may include one or more intermediate layer or surface layer interposed between the substrate main body 210 and the zeolite membrane 300. The intermediate layer or surface layer may be formed using a filtration method or flow down method using the same material as the substrate main body 210. The intermediate layer may exhibit a pore diameter that is smaller than the substrate main body 210, and the surface layer may exhibit a pore diameter that is smaller than the intermediate layer. When the substrate 200 includes the intermediate layer and surface layer, the zeolite membrane 300 is formed on the inner surface of the surface layer, and when the substrate 200 has an intermediate layer, the zeolite membrane 300 is formed on an inner surface of the intermediate layer.

(D) In the above embodiment, the zeolite membrane was formed by one continuous process of hydrothermal synthesis. However, the formation is possible by hydrothermal synthesis on a discontinuous number of occasions.

(E) In the above embodiment, zeolite powder (zeolite seed crystal) was deposited onto an inner surface of the through holes TH. However, a starting material solution including a dispersion of the zeolite powder may be used, or use of a zeolite powder may be omitted.

(F) Although the substrate main body 210 includes formation of plural through holes TH, at least one through hole TH may be formed in the substrate main body 210. That is to say, the substrate 200 is not limited to a so-called monolithic shape and may be a tube shape. The term "monolithic" denotes a shape in which plural through holes is formed in a longitudinal direction, and is a concept that includes a honeycomb shape. The zeolite membrane may be formed only on the outer surface or the inner surface of the substrate 200, or may be formed on both the inner surface and the outer surface.

(G) In the above embodiment, the separation membrane structure 100 comprises the substrate main body 210 and the zeolite membrane 300. However, the separation membrane structure 100 may only include the zeolite membrane 300.

(H) In the above embodiment, the zeolite according to the present invention is applied to the zeolite membrane 300 of the separation membrane structure 100. However, the zeolite according to the present invention may be used in a configuration as a powder in addition to a configuration as a membrane. A zeolite powder in this configuration for example may be used as an absorbent or a catalyst.

EXAMPLES

The examples of the present invention will be described below. However, the present invention is not thereby limited to the following examples.

Preparation of Samples No. 1 to No. 13

A zeolite powder according to Samples No. 1 to No. 13 is prepared as described below.

Firstly, the starting materials shown in Table 1 (silica source, alumina source, alkali source, solvent) are mixed in predetermined ratios in a fluorocarbon resin wide-mouthed jar and stirred to thereby prepare a starting material solution. The mixing ratios of the starting materials are adjusted so that the value of the Ag/(Si + 10 x Na) powder after ion exchange as described hereafter coincides with the values shown in Table 1.

After pouring the starting materials solution into a stainless steel pressure-resistant container (including an inner tube of fluorocarbon resin) having an internal volume of 100 ml, a heating process (hydrothermal synthesis) is performed under the synthesis conditions shown in Table 1.

The powder resulting from hydrothermal synthesis is washed in water and dried at 80 degrees C to thereby obtain a powder.

Next, the powder for Sample Nos. 7, 8, and 11 to 13 is placed into a furnace, and heated in an atmosphere of air (500 degrees C, 4 hours) to combust and remove the structure regulation agent.

Then, Ag ion exchange is performed by immersing the resulting powder for 24 hours in a solution containing a predetermined ratio mixture of a 0.1 mol/L aqueous solution of silver nitrate and a 0.1 mol/L aqueous solution of sodium nitrate. The mixing ratio of the aqueous solution of silver nitrate and the aqueous solution of sodium nitrate is adjusted so that the Ag/Na value coincides with the values shown in Table 1.

The powder resulting from ion exchange is washed in water and dried at 80 degrees C to thereby obtain a zeolite powder.

Next, the zeolite powder is measured using XRD to thereby identify the crystal phase (type of zeolite). The crystal phase of each sample is shown in Table 1.

The molar concentration of silica contained in each sample is measured using a gravimetric method (JIS M 8853). Then ICP emission spectrometry (ULTIMA2 manufactured by Horiba Ltd.) is used to quantify the molar concentration of other components contained in each sample. Then the respectively quantified molar concentration for each element is used to calculate the value for Ag/(Si + 10 x Na) and Ag/Na that are summarized in Table 1.

Durability Testing of Gas Separation Function

The zeolite powder in Sample Nos. 1 to 13 is used to perform durability testing on the gas separation function. In this context, absorption measurement of ethylene/ethane is performed to observe the separation function of olefin/paraffin. In the event of confirmation of a characteristic tendency for ethylene to be absorbed more than ethane, it is possible to determine that there is a high separation function for olefin/paraffin. Furthermore, changes in the ethylene/ethane absorption characteristics when exposed to a hydrogen atmosphere that promotes Ag aggregation are evaluated for the purpose of confirming the Ag aggregation inhibiting action of the zeolite powder. Since tiny Ag aggregation occurred in a normal usage environment when there was little variation in the ethylene/ethane absorption characteristics before and after exposure to a hydrogen atmosphere, it is confirmed that there is a low deterioration in the separation function of olefin/paraffin and that durability is exhibited.

Firstly, a pure ethylene gas and a pure ethane gas are respectively supplied to the zeolite powder in a 23 degree C and 0 MPa to 1 MPa environment to thereby calculate (ethylene absorption amount at 1 MPa)/(ethane absorption amount at 1 MPa).

Next, the zeolite powder is exposed to a hydrogen atmosphere by causing a pure hydrogen gas to flow at normal pressure (70 degrees C, 24 hours) into a glass tube in which the zeolite powder is disposed.

Then, a pure ethylene gas and a pure ethane gas are respectively supplied to the zeolite powder in a 23 degree C and 0 MPa to 1 MPa environment to thereby re-calculate (ethylene absorption amount at 1 MPa)/(ethane absorption amount at 1 MPa).

Olefin/paraffin gas separation is evaluated based on the calculated results for (ethylene absorption amount at 1 MPa)/(ethane absorption amount at 1 MPa) before and after hydrogen exposure. Table 1 denotes "○" as olefin selectivity in a sample evaluated as exhibiting a high separation function, "◎" as selectivity in a sample evaluated as particularly high, and "X" as selectivity in a sample evaluated as low.

The maintenance rate for the olefin/paraffin separation function before and after hydrogen exposure is calculated to thereby evaluate whether or not the olefin/paraffin separation function is maintained before and after hydrogen exposure. Table 1 denotes "○" as durability in a sample evaluated as exhibiting a high maintenance rate for the separation function, "◎" as durability in a sample evaluated as particularly high, and "X" as durability in a sample evaluated as low.

Table 1

| Sample No. | Starting Materials | | | | | Synthesis Temperature | Synthesis Time | Crystal Phase | Ag/(Si+10×Na) | Ag/Na | Selectivity | Durability |
|---|---|---|---|---|---|---|---|---|---|---|---|---|
| | Silica Source | Alumina Source | Alkali Source | Structure Regulation Agent | Solvent | | | | | | | |
| 1 | sodium silicate | sodium aluminate | sodium hydroxide | - | water | 90°C | 25h | A-type zeolite | 0,02 | 0,3 | ○ | ◎ |
| 2 | sodium silicate | sodium aluminate | sodium hydroxide | - | water | 90°C | 25h | A-type zeolite | 0,14 | 1,9 | ◎ | ◎ |
| 3 | sodium silicate | sodium aluminate | sodium hydroxide | - | water | 90°C | 25h | A-type zeolite | 0,18 | 2,4 | ◎ | × |
| 4 | sodium silicate | sodium aluminate | sodium hydroxide | - | water | 100°C | 25h | Y-type zeolite | 0,03 | 0,7 | ◎ | ◎ |
| 5 | sodium silicate | sodium aluminate | sodium hydroxide | - | water | 100°C | 25h | Y-type zeolite | 0,15 | 11,2 | ◎ | ○ |
| 6 | sodium silicate | sodium aluminate | sodium hydroxide | - | water | 100°C | 25h | Y-type zeolite | 0,17 | 32,3 | ◎ | ○ |
| 7 | colloidal silica | sodium aluminate | - | tetraethylammonium hydroxide | water | 130°C | 100h | BEA-type zeolite | 0,04 | >100 | ◎ | ◎ |
| 8 | colloidal silica | sodium aluminate | sodium hydroxide | 1-adamantane amine | water | 160°C | 15h | DDR-type zeolite | 0,03 | >100 | ◎ | ◎ |
| 9 | colloidal silica | sodium aluminate | sodium hydroxide | - | water | 120°C | 25h | MFI-type zeolite | 0,02 | 1,0 | ○ | ◎ |
| 10 | colloidal silica | sodium aluminate | sodium hydroxide | - | water | 120°C | 25h | MFI-type zeolite | 0,04 | 11,5 | ◎ | ◎ |
| 11 | colloidal silica | sodium aluminate | sodium hydroxide | tetrapropylammonium hydroxide | water | 120°C | 25h | MFI-type zeolite | 0,03 | 19,8 | ◎ | ◎ |
| 12 | colloidal silica | sodium aluminate | sodium hydroxide | tetrapropylammonium hydroxide | water | 120°C | 25h | MFI-type zeolite | 0,02 | >100 | ○ | ◎ |
| 13 | colloidal silica | sodium aluminate | - | tetrapropylammonium hydroxide | water | 120°C | 25h | MFI-type zeolite | 0,01 | >100 | × | ◎ |

As shown in Table 1, in Sample Nos. 1, 2 and 4 to 12 that satisfy the relation $0.02 \leqq Ag/(Si + 10 \times Na) \leqq 0.17$, superior results are obtained in relation to both selectivity and durability. This fact demonstrates that Ag ions are separated by an interval and that an alkali metal or alkaline earth metal is disposed between Ag ions thereby resulting in inhibition of Ag ion aggregation. This result is conspicuous in Sample Nos. 2, 4, 7, 8, 10 and 11 that satisfy the relation $0.03 \leqq Ag/(Si + 10 \times Na) \leqq 0.14$.

On the other hand, Sample No. 3 does not exhibit sufficient durability for Ag to aggregate into a high density configuration and form a metallic configuration. Furthermore, Sample No. 13 does not exhibit sufficient selectivity due to an excessively low Ag concentration.

Although not illustrated in Table 1, there is experimental confirmation of the feature that a similar effect to that exhibited by Sample Nos. 1 to 13 has also been obtained in relation to the samples undergoing Ag ion exchange after ion exchange to K or Ca.

Preparation of Sample Nos. 14 to 18

A separation membrane structure according to Samples No. 14 to No. 18 is prepared as described below.

Firstly, 20 parts by mass of an inorganic binder is added to 100 parts by mass of alumina particles having an average particle diameter of 50 micrometers, then water, a dispersing agent and a thickener are added, and the mixture is kneaded to prepare clay.

Next, a green body for the monolithic substrate that has plural through holes is prepared by extrusion molding of the clay.

Next, the green body for the monolithic substrate is fired (1250 degrees C, 1 hour). The size of the monolithic substrate is a diameter of 30 mm and a length of 15 mm. A monolithic support body is prepared by disposing an intermediate layers formed from an alumina porous body having an average pore diameter of 0.5 micrometers and an average pore diameter of 0.1 micrometers sequentially on the surface of the through holes.

Next, starting materials (silica source, alumina source, alkali source, solvent) are added to a fluorocarbon resin wide-mouthed jar and stirred to thereby prepare a starting material solution. More specifically, Sample No. 14 is prepared using the same starting material solution as Sample No. 2, Sample No. 15 is prepared using the same starting material solution as Sample No. 4, Sample No. 16 is prepared using the same starting material solution as Sample No. 7, Sample No. 17 is prepared using the same starting material solution as Sample No. 8, and Sample No. 18 is prepared using the same starting material solution as Sample No. 11.

Then, a zeolite crystal is dispersed using ethanol, and a seeding slurry liquid adjusted to have a concentration of 0.1 mass% is caused to flow into the cells of the monolithic support body. In Sample No. 14, the zeolite powder obtained by the heating process (hydrothermal synthesis) using the same method as Sample No. 2 is configured as the zeolite seed crystal, in Sample No. 15, the zeolite powder obtained by the heating process using the same method as Sample No. 4 is configured as the zeolite seed crystal, in Sample No. 16, the zeolite powder obtained by the heating process using the same method as Sample No. 7 is configured as the zeolite seed crystal, in Sample No. 17, the zeolite powder obtained by the heating process using the same method as Sample No. 8 is configured as the zeolite seed crystal, in Sample No. 18, the zeolite powder obtained by the heating process using the same method as Sample No. 11 is configured as the zeolite seed crystal.

After blow drying the interior of the cells, and after pouring the starting material solution into the into a stainless steel pressure-resistant container (including an inner tube of fluorocarbon resin) having an internal volume of 300 ml, the monolithic support body with attached seed crystals is immersed and a heating process (hydrothermal synthesis) is performed. In Sample No. 14, the heating process is performed under the same conditions as Sample No. 2, in Sample No. 15, the heating process is performed under the same conditions as Sample No. 4, in Sample No. 16, the heating process is performed under the same conditions as Sample No. 7, in Sample No. 17, the heating process is performed under the same conditions as Sample No. 8, in Sample No. 18, the heating process is performed under the same conditions as Sample No. 11.

A monolithic support body that forms a zeolite membrane is obtained by drying at 80 degrees C after washing the monolithic support body in water. Next, the monolithic support body in Sample Nos. 16 to 18 is placed into an electrical furnace, and heated in an atmosphere of air (500 degrees C, 4 hours) to combust and remove the structure regulation agent. Then, Ag ion exchange is performed in Sample Nos. 14 to 18 by use of a mixed solution containing an aqueous solution of silver nitrate and an aqueous solution of sodium nitrate to thereby form a zeolite membrane.

Next, the zeolite membrane is measured by XRD to thereby identify the crystal phase (zeolite framework structure). The crystal phase of each sample is shown in Table 2.

The inner diameter of the zeolite pores is calculated with reference to the crystal phase. The inner diameter of the pores in each sample is shown in Table 2.

Evaluation of Gas Permeability

The gas permeability of the separation membrane body according to Sample Nos. 14 to 18 is evaluated. More specifically, an ethylene/ethane mixed gas (1:1) is supplied at measurement conditions of 23 degrees C and 1 MPa, and the gas amount and component emitted from the side surface of the support body are detected. The permeability and selectivity of ethylene are evaluated based on the component and amount of detected gas.

Table 2

| Sample No. | Crystal Phase | Inner Diameter of Pore (nm) | Selectivity | Permeability |
|---|---|---|---|---|
| 14 | A-type zeolite | 0,41 | ◎ | ◎ |
| 15 | $\gamma$-type zeolite | 0,74 | ○ | ◎ |
| 16 | BEA-type zeolite | 0,66 | ◎ | ◎ |
| 17 | DDR-type zeolite | 0,36 | ◎ | ○ |
| 18 | MFI-type zeolite | 0,53 | ◎ | ◎ |

As shown in Table 2, all of Sample Nos. 14 to 18 exhibit superior selectivity and permeability for ethylene. Therefore the zeolite according to Sample Nos. 1, 2, and 4 to 12 is confirmed to be adapted to the separation membrane structure for gas separation.

As shown in Table 2, Sample Nos. 14, and 16 to 18 exhibit higher selectivity than Sample No. 15. This feature is considered to be due to the fact that the inhibiting effect on paraffin inflow into the pores by olefin is stronger since the inner diameter of the pores in the zeolite in Sample Nos. 14 and 16 to 18 is smaller than Sample No. 15. Therefore it is understood that the inner diameter of the zeolite pores is preferably no more than 0.7 nm.

Sample Nos. 14 to 16, 18 exhibit higher permeability than Sample No. 17. This feature is considered to be due to the fact that olefin tends to penetrate the pores since the inner diameter of the pores in the zeolite in Sample Nos. 14 and 16 to 18 is larger than Sample No. 17. Therefore it is understood that the inner diameter of the zeolite pores is preferably at least 0.4 nm.

DESCRIPTION OF THE REFERENCE NUMERALS

|  |  |
|---|---|
| 100 | MONOLITHIC SEPARATION MEMBRANE STRUCTURE |
| 200 | SUBSTRATE |
| 210 | SUBSTRATE MAIN BODY |
| 220 | FIRST SEAL PORTOIN |
| 230 | SECOND SEAL PORTOIN |
| 300 | ZEOLITE MEMBRANE |
| TH | THROUGH HOLES |

**Claims**

1. A zeolite comprising Si, Al, Ag and at least one of an alkali metal or alkaline earth metal, and satisfying Relation 1 below.

$$(\text{Relation 1})$$
$$0.02 \leqq \text{Ag[mol\%]}/(\text{Si[mol\%]} + 10 \times \text{T[mol\%]}) \leqq 0.17$$

(Wherein, in Relation 1, T[mol%] denotes a molar concentration of the alkali metal and alkaline earth metal.)

2. The zeolite according to claim 1 and satisfying Relation 2 below.

(Relation 2)

$$0.03 \leqq Ag[mol\%]/(Si[mol\%] + 10 \times T[mol\%]) \leqq 0.14$$

(Wherein, in Relation 2, T[mol%] denotes the molar concentration of the alkali metal and alkaline earth metal.)

3. The zeolite according to claim 1 or claim 2, wherein
a maximum value of an inner diameter of at least one of a plurality of pores is greater than or equal to 0.4 nm.

4. The zeolite according to one of claim 1 to claim 3, wherein
the maximum value of the inner diameter of the respective plurality of pores is less than or equal to 0.7 nm.

5. A separation membrane structure comprising
a porous substrate main body; and
a zeolite membrane disposed on a surface of the substrate main body; wherein
the zeolite membrane comprises Si, Al, Ag and at least one of an alkali metal or alkaline earth metal, and satisfies
Relation 1 below.

(Relation 1)

$$0.02 \leqq Ag[mol\%]/(Si[mol\%] + 10 \times T[mol\%]) \leqq 0.17$$

(Wherein, in Relation 1, T[mol%] denotes a molar concentration of the alkali metal and alkaline earth metal.)

6. The separation membrane structure according to claim 5 and satisfying Relation 2 below.

(Relation 2)

$$0.03 \leqq Ag[mol\%]/(Si[mol\%] + 10 \times T[mol\%]) \leqq 0.14$$

(Wherein, in Relation 2, T[mol%] denotes the molar concentration of the alkali metal and alkaline earth metal.)

7. The separation membrane structure according to claim 5 or claim 6, wherein
the zeolite membrane comprises a plurality of pores, and a maximum value of an inner diameter of at least one of
a plurality of pores is greater than or equal to 0.4 nm.

8. The separation membrane structure according to claim 5 to claim 7, wherein
the maximum value of the inner diameter of the respective plurality of pores is less than or equal to 0.7 nm.

9. A method of manufacturing a zeolite comprising the steps of;
preparing a zeolite by hydrothermal synthesis, the zeolite comprising Si, Al, Ag and at least one of an alkali metal
or alkaline earth metal; and
performing ion exchange on the zeolite with Ag by immersing the zeolite in a mixed solution containing at least a
silver compound and an alkali metal compound and/or an alkaline earth metal compound.

10. The method of manufacturing a zeolite according to claim 9, wherein
the zeolite after ion exchange satisfies Relation 1 below.

(Relation 1)

$$0.02 \leqq Ag[mol\%]/(Si[mol\%] + 10 \times T[mol\%]) \leqq 0.17$$

(Wherein, in Relation 1, T[mol%] denotes a molar concentration of the alkali metal and alkaline earth metal.)

**Amended claims under Art. 19.1 PCT**

**1.** A zeolite used in olefin/paraffin separation, the zeolite comprising Si, Al, Ag and at least one of an alkali metal or alkaline earth metal, and satisfying Relation 1 below.

$$\text{(Relation 1)}$$
$$0.02 \leqq \text{Ag[mol\%]}/(\text{Si[mol\%]} + 10 \times \text{T[mol\%]}) \leqq 0.17$$

(Wherein, in Relation 1, T[mol%] denotes the molar concentration of the alkali metal and alkaline earth metal.)

**2.** The zeolite according to claim 1 and satisfying Relation 2 below.

$$\text{(Relation 2)}$$
$$0.03 \leqq \text{Ag[mol\%]}/(\text{Si[mol\%]} + 10 \times \text{T[mol\%]}) \leqq 0.14$$

(Wherein, in Relation 2, T[mol%] denotes the molar concentration of the alkali metal and alkaline earth metal.)

**3.** The zeolite according to claim 1 or claim 2,wherein
a maximum value of an inner diameter of at least one of a plurality of pores is greater than or equal to 0.4 nm.

**4.** The zeolite according to one of claim 1 to claim 3, wherein
the maximum value of the inner diameter of the respective plurality of pores is less than or equal to 0.7 nm.

**5.** A separation membrane structure used in olefin/paraffin separation, the separation membrane structure comprising
a porous substrate main body; and
a zeolite membrane disposed on a surface of the substrate main body; wherein
the zeolite membrane comprising Si, Al, Ag and at least one of an alkali metal or alkaline earth metal, and satisfying Relation 1 below.

$$\text{(Relation 1)}$$
$$0.02 \leqq \text{Ag[mol\%]}/(\text{Si[mol\%]} + 10 \times \text{T[mol\%]}) \leqq 0.17$$

(Wherein, in Relation 1, T[mol%] denotes a molar concentration of the alkali metal and alkaline earth metal.)

**6.** The separation membrane structure according to claim 5 and satisfying Relation 2 below.

$$\text{(Relation 2)}$$
$$0.03 \leqq \text{Ag[mol\%]}/(\text{Si[mol\%]} + 10 \times \text{T[mol\%]}) \leqq 0.14$$

(Wherein, in Relation 2, T[mol%] denotes the molar concentration of the alkali metal and alkaline earth metal.)

**7.** The separation membrane structure according to claim 5 or claim 6, wherein
the zeolite membrane comprises a plurality of pores, and a maximum value of an inner diameter of at least one of a plurality of pores is greater than or equal to 0.4 nm.

**8.** The separation membrane structure according to claim 5 to claim 7, wherein
the maximum value of the inner diameter of the respective plurality of pores is less than or equal to 0.7 nm.

**9.** A method of manufacturing a zeolite comprising the steps of;
preparing a zeolite by hydrothermal synthesis, the zeolite comprising Si, Al, Ag and at least one of an alkali metal or alkaline earth metal; and
performing ion exchange on the zeolite with Ag by immersing the zeolite in a mixed solution containing at least a silver compound and an alkali metal compound and/or an alkaline earth metal compound, wherein the zeolite after ion exchange satisfies Relation 1 below.

(Relation 1)

$$0.02 \leqq Ag[mol\%]/(Si[mol\%] + 10 \times T[mol\%]) \leqq 0.17$$

(Wherein, in Relation 1, T[mol%] denotes a molar concentration of the alkali metal and alkaline earth metal.)

(Relation 1)

$$0.02 \leqq Ag[mol\%]/(Si[mol\%] + 10 \times T[mol\%]) \leqq 0.17$$

LONGITUDINAL DIRECTION

FIG. 1

FIG. 2

FIG. 3

EP 3 127 866 A1

FIG. 4

FIG. 5

**EP 3 127 866 A1**

| | | |
|---|---|---|
| **INTERNATIONAL SEARCH REPORT** | International application No. | PCT/JP2015/058449 |

**A. CLASSIFICATION OF SUBJECT MATTER**
*C01B39/16*(2006.01)i, *B01D69/12*(2006.01)i, *B01D71/02*(2006.01)i, *B01J20/18*
(2006.01)i, *B01J20/30*(2006.01)i, *C01B39/24*(2006.01)i, *C01B39/38*(2006.01)i,
*C01B39/40*(2006.01)i, *C01B39/48*(2006.01)i
According to International Patent Classification (IPC) or to both national classification and IPC

**B. FIELDS SEARCHED**

Minimum documentation searched (classification system followed by classification symbols)
C01B39/16, B01D69/12, B01D71/02, B01J20/18, B01J20/30, C01B39/24,
C01B39/38, C01B39/40, C01B39/48

Documentation searched other than minimum documentation to the extent that such documents are included in the fields searched
Jitsuyo Shinan Koho     1922–1996    Jitsuyo Shinan Toroku Koho    1996–2015
Kokai Jitsuyo Shinan Koho    1971–2015    Toroku Jitsuyo Shinan Koho    1994–2015

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>A | JP 2005-520680 A (Council of Scientific and Industrial Research),<br>14 July 2005 (14.07.2005),<br>claims; examples 12 to 14<br>& JP 2003-275582 A    & US 6572838 B1<br>& GB 2386889 A    & GB 207012 D0<br>& WO 2003/080236 A1    & DE 60323065 D<br>& AU 2003255490 A    & CN 1668374 A<br>& RU 2004131543 A | 1-8<br>9,10 |
| X<br>A | JP 10-095611 A (Nippon Sanso Corp.),<br>14 April 1998 (14.04.1998),<br>claims; example 1<br>(Family: none) | 1-8<br>9,10 |

☒ Further documents are listed in the continuation of Box C.    ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| | |
|---|---|
| Date of the actual completion of the international search<br>11 June 2015 (11.06.15) | Date of mailing of the international search report<br>23 June 2015 (23.06.15) |
| Name and mailing address of the ISA/<br>Japan Patent Office<br>3-4-3,Kasumigaseki,Chiyoda-ku,<br>Tokyo 100-8915,Japan | Authorized officer<br><br>Telephone No. |

Form PCT/ISA/210 (second sheet) (July 2009)

**INTERNATIONAL SEARCH REPORT**

| International application No. |
| --- |
| PCT/JP2015/058449 |

C (Continuation).   DOCUMENTS CONSIDERED TO BE RELEVANT

| Category* | Citation of document, with indication, where appropriate, of the relevant passages | Relevant to claim No. |
| --- | --- | --- |
| X<br>A | JP 10-095612 A  (Nippon Sanso Corp.),<br>14 April 1998 (14.04.1998),<br>claims; examples<br>(Family: none) | 1-3,5-7<br>4,8-10 |
| X<br>A | JP 8-245326 A  (Kanebo, Ltd.),<br>24 September 1996 (24.09.1996),<br>examples 7 to 9<br>& US 6071542 A          & GB 2315675 A<br>& WO 1996/028028 A1 | 9<br>1-8,10 |
| X<br>A | JP 4-224505 A  (The Nippon Chemical Industrial<br>Co., Ltd.),<br>13 August 1992 (13.08.1992),<br>examples; table 2<br>(Family: none) | 9<br>1-8,10 |
| X<br>A | JP 4-021517 A  (The Nippon Chemical Industrial<br>Co., Ltd.),<br>24 January 1992 (24.01.1992),<br>examples; table 2<br>(Family: none) | 9<br>1-8,10 |
| X<br>A | JP 4-202010 A  (Kanebo, Ltd.),<br>22 July 1992 (22.07.1992),<br>examples<br>& US 5206195 A          & DE 4117964 A<br>& CA 2043692 A1 | 9<br>1-8,10 |

Form PCT/ISA/210 (continuation of second sheet) (July 2009)

## INTERNATIONAL SEARCH REPORT

| International application No. |
| --- |
| PCT/JP2015/058449 |

**Box No. II      Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:
       The technical feature of the invention of claim 1 has been publicly known as disclosed in the documents 1-3.
       Consequently, claims 1-10 involve two or more invention groups, since said claims 1-10 are classified into a main invention group of claims 1-4 and invention groups of other claims.
       Document 1: JP 2005-520680 A  (Council of Scientific and Industrial Research), 14 July 2005 (14.07.2005)
       Document 2: JP 10-095611 A  (Nippon Sanso Corp.), 14 April 1998 (14.04.1998)
       Document 3: JP 10-095612 A  (Nippon Sanso Corp.), 14 April 1998 (14.04.1998)

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**      ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **MASAHIKO MATSUKATA et al.** Gin Kachion Kokankei Y-kei Zeoraitomaku ni yoru Puropan/Puropiren Bunri no Kanosei. *SCEJ (The Society of Chemical Engineers Japan) 78th Annual Meeting Q109,* 17 March 2013, URL:http://www3.scej.org/meeting/78a/prog/sess_22.html

- *Database of Zeolite Structures,* 13 February 2015, http://www.iza-structure.org/databases